# EUROPEAN PATENT APPLICATION

(11) **EP 3 326 580 A1**
(43) Date of publication of application: **30.05.2018**
(21) Application number: 16200959.1
(22) Date of filing: 28.11.2016
(51) Int. Cl.: A61C 19/06, A61C 17/02, A61L 2/18

(54) **MEDICAL DEVICE FOR INTRAORAL USE**

(30) Priority: 26.11.2016 IT 201500077378; 26.11.2016 IT 201500077401
(71) Applicant: Delle Grazie, Massimo, 40018 San Pietro in Casale (Bologna) (IT)
(72) Inventor: Delle Grazie, Massimo, 40018 San Pietro in Casale (Bologna) (IT)
(74) Representative: Minghetti, Mauro

(57) **Abstract**

A medical device for intraoral use, comprising at least one oxygen concentrator (C) which is connected to at least one dispenser (2) of a gaseous mixture enriched with oxygen, said dispenser (2) comprising at least one dispensing head (3) adapted to be positioned in proximity to a pre-established zone of the surface of the oral cavity of the patient. The device comprises means (B) for dispensing at least one liquid within the gaseous mixture enriched with oxygen, so as to spray said pre-established zone with said gaseous mixture carrying said nebulized liquid, said liquid comprising one or more active principles having disinfectant, therapeutic and/or nutritional properties.

## Description

### TECHNICAL FIELD OF THE INVENTION

The present invention regards a medical device for intraoral use.

### STATE OF THE PRIOR ART

For some time, it has been known that oxygen, in specific conditions, can function as an actual drug, due to the intrinsic anti-inflammatory, anti-infective and cicatrizing properties.

In addition, it is known that the oxygen molecule has an important role in cellular metabolism, in the defense of the host and in communication between the cells. The three main treatments, described in the scientific literature, based on the clinical use of oxygen, are the following: treatment with hyperbaric oxygen, treatment with topical oxygen and treatment with continuous oxygen diffusion. Many scientific articles demonstrate the beneficial effects and principles of action of the various treatments with oxygen.

One of the most important applications of oxygen in therapeutic, hospital and underwater contexts is hyperbaric oxygen therapy, through which it is possible to treat/accelerate the curative processes of a long series of different pathologies. More in detail, it was observed that, during tissue repair and wound healing processes, the request for oxygen increases, so that today all clinical efforts are concentrated towards the correction of the hypoxia condition, i.e. insufficient presence of oxygen.

Based on such knowledge, over time different applications were designed and developed that exploit that dispensing of oxygen, preferably with high purity level and at a specific pressure, even higher than atmospheric pressure, for the treatment of specific tissues (therapy with topical hyperbaric oxygen).

The most widespread applications known today mainly regard skin treatment, e.g. treatment of the face, or of other body parts in order to contrast wrinkles, spots, scars, acne, or other skin defects, restoring softness and elasticity thereto, hence in general improving their appearance.

In addition to the treatments that mainly have the object of improving the health of the patient's skin, and hence which have mainly aesthetic aim, there are also other treatments that exploit the dispensing of oxygen on the skin or on mucous membranes in order to contrast specific pathologies or infections, accelerate the healing of wounds, and the like.

For example, the United States patent US 5,928,187 describes a device for prophylaxis and treatment of gum diseases which exploits the dispensing of oxygen.

The device described in this document comprises a dispenser, with complex shape, that follows the entire dental arch and dispenses oxygen directly at teeth and gums; the dispenser comprises sealing means which, abutting against the gums themselves, allow maintaining the correct pressure of the dispensed oxygen. Such device is however structurally complex and very inconvenient to use, poorly adapted to the dental arches of different shape.

Moreover, the operator has poor control over the dispensing in the various zones of the arch, since the presence of sealing means prevents the verification of correct process execution, or the verification that all the affected zones have been effectively treated with direct and timely spraying of oxygen.

This poor controllability of the process can very negatively affect the execution times as well, since it may frequently be necessary to repeat the treatment in the zones that have not been suitably sprayed; it follows that the work of the professional cannot be effective as would be expected.

It must be added that the device described in this document is highly uncomfortable and invasive also for the patient himself/herself.

### OBJECTS OF THE INVENTION

The technical aim of the present invention is to improve the state of the art.

Within such technical aim, one object of the present invention is to implement a medical device for intraoral use that allows carrying out the treatment of various pathologies of the oral cavity in a more effective manner than what has been possible to achieve with the currently known means.

Still another object of the present invention is to provide a medical device for intraoral use that allows carrying out the treatment of various pathologies of the oral cavity in a more precise and accurate manner with respect to what can be done with the devices of known type, in particular with increased possibility of control and verification.

A further object of the present invention is to make a medical device for intraoral use that allows carrying out the treatment of various pathologies of the oral cavity in a quicker manner than what can be done with the known means.

Such aim and such objects are all achieved by the medical device for intraoral use according to the enclosed claim 1.

The medical device for intraoral use comprises at least one oxygen concentrator, which is connected to at least one dispenser of a gaseous mixture enriched with oxygen; such dispenser comprises at least one dispensing head adapted to be positioned in proximity to a pre-established zone of the surface of the oral cavity of the patient.

According to the invention, the device comprises means for dispensing at least one liquid within the gaseous mixture enriched with oxygen, so as to spray said pre-established zone with the gaseous mixture carrying the nebulized liquid; the liquid comprises one or more active principles having disinfectant, therapeutic and/or nutritional properties.

In one embodiment of the invention of particular interest, the aforesaid liquid comprises povidone-iodine and/or chlorhexidine.

In one embodiment of the invention, the aforesaid liquid comprises vitamin complexes.

The dependent claims refer to advantageous embodiments of the invention.

### BRIEF DESCRIPTION OF THE DRAWINGS

These and further advantages will be better understood by each man skilled in the art from the following description and the enclosed drawings, given as a non-limiting example, in which:
figure 1 is a perspective and schematic view of the device according to the invention;
figure 2 is a schematic detail side view of the dispenser of the device;
figure 3 is a partially sectioned side view of the dispenser of the device in one embodiment of the invention;
figure 3A is a detail of figure 3;
figure 4 is a side view of the dispenser of the device in another embodiment of the invention;
figure 5 is a side view of the dispenser of the device in yet another embodiment of the invention;
figure 6 is a perspective view of the dispensing terminal of the device, in another embodiment of the invention;
figure 7 is a perspective view of the dispensing terminal in another embodiment of the invention;
figure 8 is a perspective view of the dispensing terminal in yet another embodiment of the invention;
figure 9 is a plan view of the dispensing terminal of figure 8;
figure 10 is a perspective view of the dispensing terminal in yet another embodiment of the invention;
figure 11 is a side view of the dispensing terminal of figure 10;
figure 12 is a perspective view of the dispensing terminal in a further embodiment of the invention;
figure 13 is a perspective view of the dispensing terminal in a further embodiment of the invention;
figure 14 is a perspective view of the dispensing terminal in yet another embodiment of the invention;
figure 15 is a perspective view of the dispensing terminal in a further embodiment of the invention.

### EMBODIMENTS OF THE INVENTION

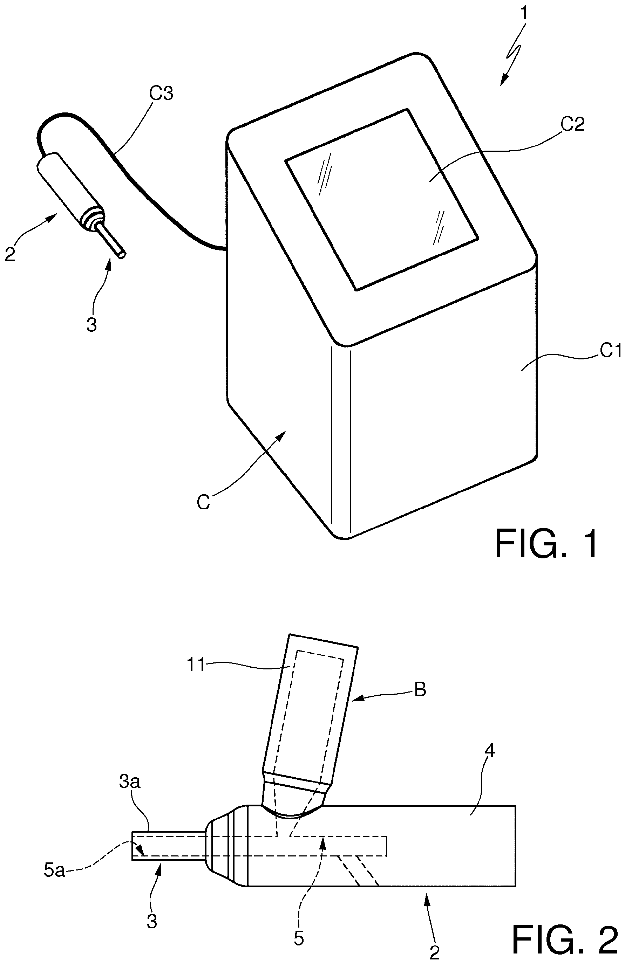

With reference to figure 1, reference number 1 overall indicates, only for illustrating and non-limiting purposes, a medical device for intraoral use according to the present invention.

The device 1 comprises at least one oxygen concentrator C, shown in an entirely schematic manner and only for illustrating purposes.

The oxygen concentrator C is capable of producing a specific flow rate of a mixture enriched with oxygen, using the ambient air as main source.

In the course of the present description, with the expression mixture, or gaseous mixture, or gaseous mixture enriched with oxygen, it is intended a mixture enriched with oxygen produced by the aforesaid oxygen concentrator C and containing a percentage of oxygen of at least 90% by weight.

The oxygen concentrator C can for example be of so-called PSA (pressure swing adsorption) type or of another equivalent type, without limitations for the objects of the present invention.

The oxygen concentrator C comprises a box-like support structure C1.

In addition, the oxygen concentrator C comprises a user interface C2, provided with means for driving and controlling the main functions of the device 1.

The oxygen concentrator C also comprises a duct C3 for feeding the mixture enriched with oxygen.

The device 1 comprises a dispenser 2 for the mixture enriched with oxygen; the dispenser 2 is connected to the duct C3.

In figure 1, the dispenser 2 is shown in an entirely schematic manner; the characteristics of the dispenser 2 are better illustrated in the subsequent figures, and in the following description.

The dispenser 2 comprises at least one dispensing head 3.

The dispensing head 3 is adapted to be positioned in proximity to a pre-established zone of the surface of the oral cavity of the patient.

The dispensing head 3 is suitable for directed, over such pre-established zone, at least one jet comprising the aforesaid gaseous mixture enriched with oxygen, as will be better described hereinbelow.

The dispenser 2 can also comprise at least one grip portion 4, for example with ergonomic conformation or with other characteristics suitable for facilitating the use thereof by the operator.

The dispenser 2 comprises at least one channel 5, which crosses it.

The channel 5 is placed in communication with the duct C3 for feeding the gaseous mixture enriched with oxygen.

The channel 5 terminates, with the respective outlet opening 5a, in the dispensing head 3, and from here towards the outside.

The head 3 comprises an end 3a with a through cavity.

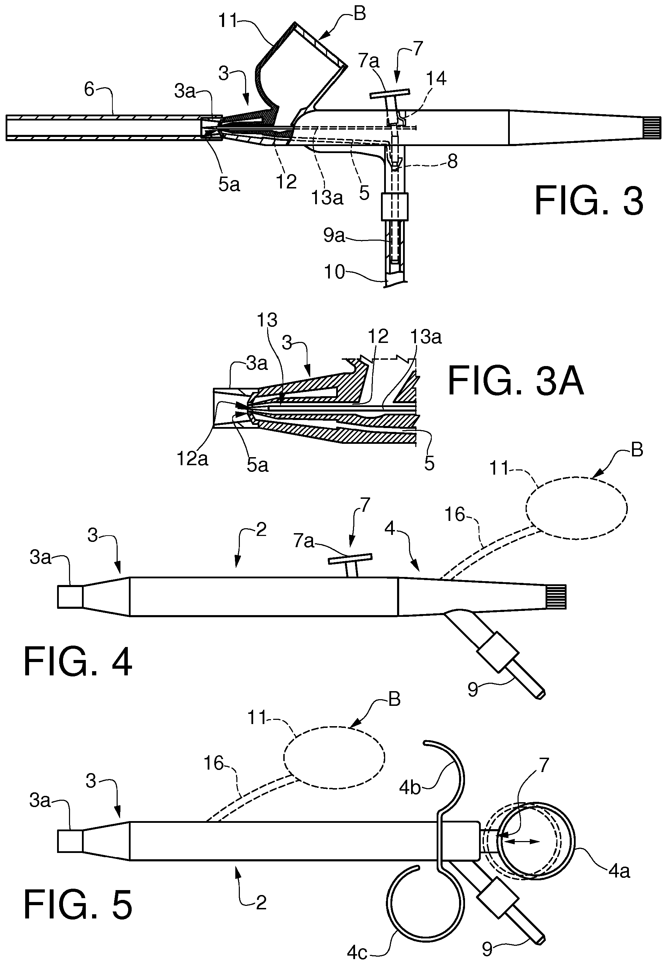

In the particular embodiment of the dispenser 2 illustrated in figure 3, the cavity of the end 3a of the head 3 preferably has frustoconical shape, with smaller diameter directed towards the interior.

In the embodiment of figure 3, the head 3 can also comprise - optionally - at least one dispensing cannula 6.

Such cannula 6 can be removably coupled to the head 3, and in particular to its terminal portion 3a.

The coupling can be carried out, for example, via interference or by means of respective threaded portions that are mutually engaged, or with a quick coupling of bayonet type, or the like.

Alternatively, the cannula 6 can be integrally made with the end 3a of the head 3. The cannula 6 can be rigid, or it can be flexible/deformable so as to maintain the shape that the operator manually confers thereto, in order to better treat the surfaces of the oral cavity that would otherwise be difficult to reach.

The dispenser 2 comprises an adjustment device 7 for controlling the flow of the gaseous mixture.

The adjustment device 7 for example comprises a button 7a, which controls a shutter 8; the shutter 8 in turn intercepts the channel 5.

The operator pressing the button 7a causes the movement of the shutter 8, the opening of the channel 5 and then the flow of the gaseous mixture through the head 3; the release of the button 7a instead causes the closing of the channel 5 and the interruption of the flow of the gaseous mixture.

An alternative solution provides that the button 7a can be selectively locked in a plurality of intermediate positions between that of opening and that of closing, in order to determine the flow of gaseous mixture with a variable flow rate.

The dispenser 2 comprises a tubular appendage 9, within which an inlet duct 9a for the gaseous mixture is provided.

The inlet duct 9a communicates with the channel 5; in turn, the inlet duct 9a communicates with the terminal end 10 of the duct C3 for feeding the gaseous mixture.

According to one aspect of the invention, the device 1 comprises means B for dispensing at least one liquid within the gaseous mixture enriched with oxygen.

More in detail, the dispensing means B are adapted to release, within the gaseous mixture enriched with oxygen, a predetermined flow of liquid, which is then nebulized and carried towards the affected zone due to the action of the same gaseous mixture.

The liquid can be constituted by a mixture comprising one or more active principles having specific disinfectant, therapeutic and nutritional properties, as well as other properties, as will be better described hereinbelow.

According to another aspect of the invention, the aforesaid liquid comprises povidone-iodine and/or chlorhexidine.

In particular, a diluted solution of povidone-iodine and/or chlorhexidine can be used.

In one embodiment of the invention, the aforesaid liquid can comprise Betadine®, i.e. a 10% povidone-iodine solution.

In one embodiment of the invention, the aforesaid liquid can comprise galenical chlorhexidine, and hence lack excipients: this allows reducing the risks of sensitization of the treated tissue.

At the time of treatment, the starting solution of galenical chlorhexidine is diluted with physiological solution, and then its concentration at the time of treatment is comprised between 0.1% and 0.5%, and in particular can be 0.2%.

In addition, the aforesaid liquid can comprise, merely by way of a non-limiting example, vitamin complexes, or other nutrient substances.

According to the invention, the synergistic action between the gaseous mixture enriched with oxygen and the aforesaid liquid facilitates and makes more effective the penetration, in the treated tissues and/or mucous membranes, of the active principles contained in the liquid itself, and in particular of the povidone-iodine and/or chlorhexidine.

Therefore, a kind of shower is created that is capable of reaching zones normally not accessible with the normal techniques, for examples interstices, cavities, and other areas.

Indeed, the topical application of oxygen on the affected zones creates a miniature hyperbaric chamber at the affected zone: this causes a penetration of oxygen to a depth of 2-3 mm, generating an increase of the epidermal saturation of oxygen, which in turn accelerates the absorption of other infused molecules (i.e. those which constitute the aforesaid liquid).

According to the prior art, the disinfectant can be applied on the affected zones by means of a gauze, or other similar means.

The disinfectant thus applied normally penetrates into the surface layer of the epithelium for about 25 µm, with decreasing concentration gradient.

According to the present invention, the disinfectant carried by the oxygen dispensed by the concentrator C is able to penetrate the epithelium, as well as the connective tissue, up to a depth definitely greater than that reachable with the application with gauze, or the like.

It has in fact been verified that the depth reached by the disinfectant can also be at least five times greater than that reachable with the application with gauze: a considerable increase in the treatment effectiveness follows, since its action range is decidedly enlarged.

In one embodiment of the invention of particular practical interest, the dispensing means B comprise at least one tank 11; the liquid to be dispensed within the gaseous mixture is contained in the tank 11.

The tank 11 can be integral with the dispenser 2, as shown in the embodiments according to figures 2,3,3A; alternatively, the tank 11 can be physically separated from the dispenser 2 (see for example the embodiments according to the figures 4,5, which will be described in detail hereinbelow).

As shown in figure 3, the tank 11 has an opening (e.g. a lower opening, during use) which can be placed in communication with the channel 5 for feeding the gaseous mixture.

More in detail - see in particular figure 3A - the opening of the tank 11 communicates with a duct 12 in turn terminating with a hole 12a that opens into the head 3 at the outlet opening 5a of the channel 5: in this manner, the fluid communication between the channel 5 and the duct 12 is obtained.

Members 13 for controlling the patency of the hole 12a are provided, in order to control the flow rate of liquid dispensed within the gaseous mixture.

Such members 13 for controlling the patency of the hole 12a can be driven by the same button 7a, which for such purpose is movable from a closed position to a position of passage of the liquid through the hole 12a, in a manner such that the liquid can be nebulized within the flow of gaseous mixture.

The control members 13 can for example comprise a needle 13a adapted to selectively intercept the hole 12a and whose position, as stated, can be controlled by the button 7a.

When the button 7a is in the closed position, the conical tip of the needle 13a obstructs the hole 12a.

The operator brings the button 7a into the position allowing free passage of the liquid, by moving it away from the head 3: in such a manner, the needle 13a retreats and causes the opening of the hole 12a, and the consequent outflow of the liquid (as shown in figure 3A).

Automatic return means 14 are also provided for the button 7a, for automatically returning it to a closed position; such means are for example of elastic type.

Fine adjustment means of the needle 13a position may also be provided, e.g. comprising a ring nut 15 manually actuatable by the operator.

With regard to whether the dispenser 2 comprises a cannula 6 or not, the tank 11 can be associated with the dispenser 2 itself in proximity to the head 3, as in the embodiments of figures 2,3, or more spaced from the head 3 itself, such that it does not obstruct the insertion of the head 3 in the oral cavity of the patient.

In other embodiments of the device 1 (e.g. those illustrated in figures 4,5), the tank 11 is external, i.e. unconstrained by the dispenser 2, and is connected to the latter by means of at least one tube 16; the tube 16 can be rigid or flexible.

Means can also be provided for pumping the liquid from the external tank 11 towards the dispenser 2.

In figure 5, an embodiment of the invention is illustrated in which the grip portion 4 comprises ergonomic housings 4a, 4b, 4c substantially shaped as a closed and/or open ring for the insertion of the operator's finger.

Preferably the grip portion 4 comprises a first ergonomic housing 4a shaped as a closed ring and situated opposite the dispensing head 3, a second lateral ergonomic housing 4b substantially shaped as a half-ring and situated opposite a third lateral ergonomic housing 4c substantially shaped as an open or closed ring.

In this embodiment, the adjustment device 7 for controlling the dispensing of the gaseous mixture is driven by means of the first ergonomic housing 4a.

The dispenser 2 can also comprise a on/off switch (not shown in the figures).

The dispenser 2 is made of a suitable inert, non-allergenic and non-toxic material; it can be washable and/or self-washable, such that it can be reused for subsequent patient treatments.

The dispenser 2 or parts thereof, such as the cannula 6 if provided, can be of disposable type.

The operation of the device 1 according to the invention is quite evident.

The oxygen concentrator C is suitably driven, and all the main operating parameters are adjusted.

The operator then grasps the dispenser 2 and positions it with the dispensing head 3, or the cannula 6, inserted in the buccal cavity of the patient at the specific zone to be treated.

After having positioned the head 3, or the cannula 6, at the treated zone, the operator acts on the adjustment device 7 in order to dispense the desired flow rate of gaseous mixture, and he/she also acts on the control members 13 in order to allow the liquid contained in the tank 11 to be nebulized by the same gaseous mixture and then be conveyed (with the desired flow rate) towards the zone to be treated, driven by the gaseous mixture itself.

The device 1 allows carrying, on the desired surfaces of the oral cavity of the patient, a gaseous mixture enriched with oxygen with purity fractions preferably equal to 90-95% and with pressures preferably comprised between 1 atmosphere and 1.6 atmospheres, carrying the aforesaid liquid therewith.

More in detail, the more superficial treatments are preferably carried out in normal bar conditions (about 1 atm), while those which must reach deeper portions of the mucous membrane can be carried out in slightly hyperbaric conditions (about 1.6 atm).

It is provided that the dispensed flow rate can be adjusted by the operator up to a maximum of 5 liters/minute, depending on the treatment to be carried.

It is provided that the duration of the dispensing is variable in accordance with the specific treatment to be carried out in the oral cavity of the patient.

The operator can easily and precisely handle the dispenser 2 so as to effectively treat the various zones of the intraoral mucous membranes which require treatment. The mucous membranes that cover the oral cavity are susceptible of topical treatment with oxygen.

The preferred therapeutic use of the device 1 according to the present invention regards the treatment of pathological or clinically overt conditions of the mucous membranes of the buccal cavity, for prevention and maintenance of the healthy metabolic and physiological processes of the intraoral tissues.

The simultaneous use of liquid solutions of substances that are functional or pharmacologically suitable for the therapeutic purpose allows amplifying the unique action of oxygen which, above all, facilitates the carrying of hydrophilic substances by increasing the permeability of the treated tissues.

In addition, the device 1 can be used for achieving an adjuvant therapy for bacterial and fungal infections of the surface of the mucous membranes of the buccal cavity. More generally, the device 1 can be employed in all the situations in which there is evidence of the effects of oxygen, and/or in other pathological or clinically overt conditions where the treatment can bring benefit, and/or in those conditions where conventional therapy does not have the desired effects, while oxygen could potentially bring such effects.

It is also observed that the very low flows and volumes of gases employed for the treatment do not involve exposure to particular risks, since the environment is not saturated with the gaseous mixture under discussion. In any case, the same precautions for the generic use of such gases will be actuated.

The dispensing head 3 of the dispenser 2 can be interchangeable, in order to meet different operating requirements.

For this purpose, the dispensing head 3 can comprise at least one dispensing terminal to be positioned directly in proximity to the zones to be treated.

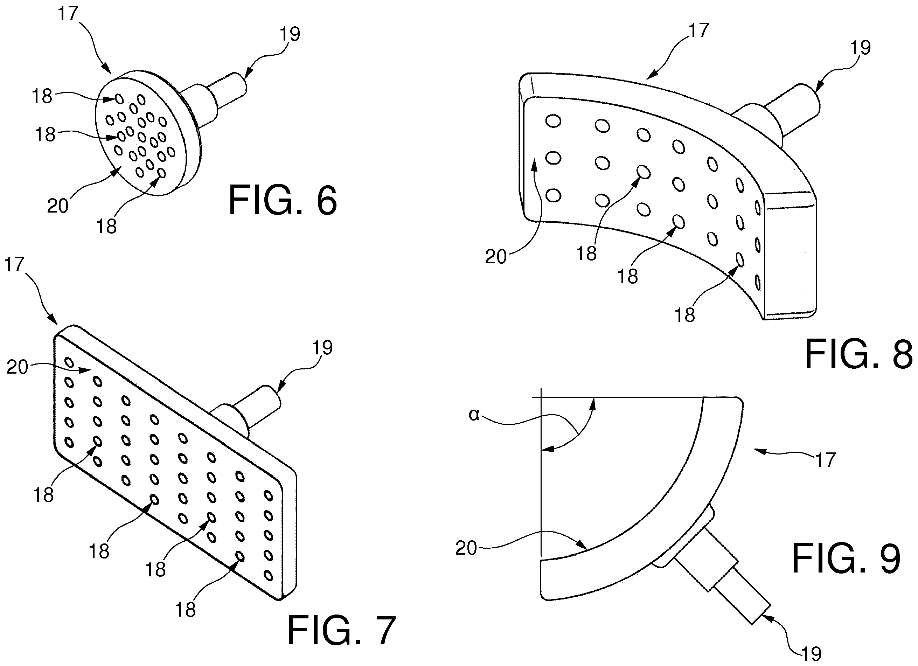

In figures 6-15, possible shapes of the dispensing terminal 17 of the head 3 are shown, according to respective embodiments of the invention.

More in detail, in figures 6-15, possible alternative shapes of the dispensing terminal 17 are shown, specifically designed for increasing the dispensing surface, and consequently reducing the times necessary for carrying out the treatment.

In the embodiment of figure 6, the dispensing terminal 17 is substantially shaped as a disc, or another similar shape.

The dispensing terminal 17 comprises a plurality of dispensing holes 18, communicating with the inlet mouth 19 of the terminal 17 itself.

The dispensing holes 18 can have uniform distribution or even non-uniform distribution, i.e. concentrated at specific zones of the dispensing surface 20 of the terminal 17.

The diameter of the dispensing holes 18 is suitably sized so as to ensure the optimal dispensing conditions - in particular with regard to the pressure and speed of the mixture gas-liquid - and also in a manner so as to avoid possible obstructions.

The discoid or substantially discoid shape of the dispensing terminal 17 constitutes a good compromise between the contrasting needs of increasing the dispensing surface 20 and of ensuring a sufficient accessibility to all zones of the oral cavity (e.g. the gums).

In the embodiment of figure 7, the dispensing terminal 17 is substantially shaped as a plate, rectangular or even with another similar form.

Also in this case, the dispensing holes 18, communicating with the inlet mouth 19 of the terminal 17, can be distributed in a uniform manner, or they can be more concentrated in specific zones of the dispensing surface 20.

This embodiment allows further increasing the dispensing surface 20, at the expense of a more difficult accessibility in specific zones of the oral cavity: the terminal 17 according to the present embodiment can then be employed in the situations where it is necessary to treat an extended, easily-accessible surface, so as to minimize the time required.

In the embodiment of figures 8,9, the dispensing terminal 17 has substantially the shape of a cylindrical crown portion, which is extended for a specific opening angle α, as shown in the plan view of figure 9.

Also in this embodiment the dispensing holes 18, communicating with the inlet mouth 19 of the terminal 17, can be distributed in a uniform manner, or they can be more concentrated in specific zones of the dispensing surface 20.

This embodiment, due to the shape of the dispensing terminal 17, allows simultaneously treating - for example in the case of gum therapy - substantially the entire surface corresponding to a half dental arch, without having to modify the position of the dispenser 2 if not for changing the half-arch to be treated.

The dispensing terminal 17 can also have a particularly ergonomic shape, in a manner such that the dispensing surface 20 is situated as close as possible to the zones to be treated.

The opening angle α can be, for example, 90° or a value close to 90°; in some embodiments of the invention the opening angle α can have a different value, for example greater than 90° or less than 90°, in a manner so as to meet different operating requirements.

By way of example, the opening angle α can be comprised between 60° and 120°.

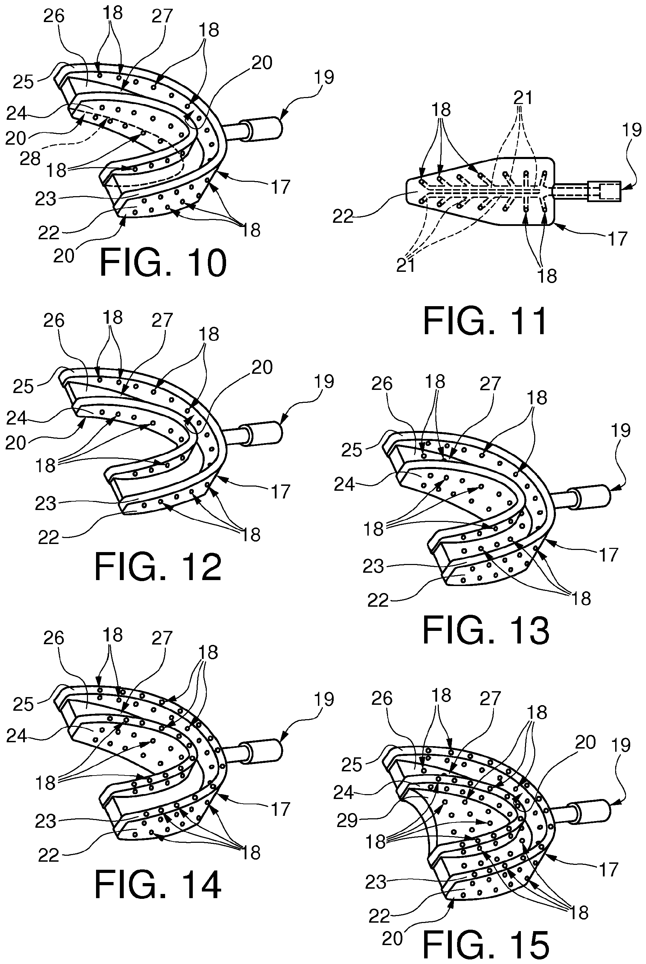

In figures 10-15, further possible alternative shapes of the dispensing terminal 17 are shown, according to respective embodiments of the invention, particularly suitable for the treatment of gums, palate, tongue and surrounding zones.

More in detail, in the embodiments of figures 10-15, the dispensing terminal 17 has shape suitable for being retained between the teeth of the patient.

In other words, the dispensing terminal 17 is substantially shaped as a dental bite, in a manner so as to allow the treatment of the entire surface of the gum mucous membrane of a single dental arch.

The dispensing terminal 17 is covered with a plurality of through channels 21 terminating in respective dispensing holes 18, distributed on the dispensing surface 20.

All the through channels 21 communicate with the inlet mouth 19 of the dispensing terminal 17.

With particular reference to the embodiment of figures 10,11, the dispensing terminal 17 comprises a first external wall 22 having at least one first edge 23, and a second internal wall 24 having at least one second edge 25.

The first wall 22 and the second wall 24 are curved, and connected by an intermediate portion 26 so as to define at least one seat 27,28 for at least one dental arch of the patient.

More particularly the first wall 22 and the second wall 24 define, with the intermediate portion 26, a first seat 27 and a second seat 28 opposite each other, respectively for the housing of the upper dental arch and the lower dental arch of the patient.

In the embodiment of figures 10,11, the dispensing surface 20 comprises in particular the internal and external surfaces of the first wall 22 and of the second wall 24, such that the dispensing of the gaseous mixture together with the liquid mainly affects the gums, though also other surfaces of the mucous membrane of the buccal cavity.

The embodiment of figure 12 is differentiated from that of the figures 10,11 in that the first wall 22 and the second wall 24 define, together with the intermediate portion 26, only the first seat 27, so as to only treat the upper dental arch or lower dental arch of the patient.

Correspondingly, the first wall 22 and the second wall 24 have lower height than that of the terminal 17 of the embodiment of figures 10,11.

The embodiment of figure 13 differs from that of figures 10,11 in that the dispensing holes 18 are also distributed on the upper and/or lower external surface of the intermediate portion 26.

Due to this expedient, it is possible to carry out the dispensing of the gaseous mixture, together with the liquid, also from a further and different direction, so as to more easily reach specific zones of the gums.

The embodiment of figure 14 differs from that of figures 10,11 in that the dispensing holes 17 are also distributed along the first edge 23, upper and/or lower, of the first wall 22, and also along the second edge 25, upper and/or lower, of the second wall 24.

This allows carrying out the treatment of the gums, or of the surrounding mucous membranes, with dispensing of gaseous mixture, mixed with liquid, which occurs according to further different directions, for the purpose of reaching still other zones of the mucous membrane of the buccal cavity.

The embodiment of figure 15 differs from that of figures 10,11 in that the dispensing terminal 17 comprises a shaped portion 29.

The shaped portion 29 is associated with the second wall 24 on the side opposite where the first wall 22 is situated.

The shaped portion 29 is affected by dispensing holes 18 distributed at least on its upper surface, in order to allow the dispensing of the gaseous mixture mixed with liquid in particular on the palate, and is curved upward so as to follow on the upper part the shape of the palate itself and house the tongue on its lower part.

In one variant, the dispensing holes 18 can also, or only, be distributed on the lower surface of the shaped portion 29, in a manner such that the dispensing also, or only, affects the tongue.

The dispensing terminal 17 can be made of a suitable biocompatible, non-allergenic, non-toxic and inert material.

The dispensing terminal 17 can be made in various sizes and shapes, in order to be adapted to the characteristics of the patient.

The dispensing terminal 17 can also be of self-modeling type, for example made of a thermoformable material so that, heated in hot water at first use, it is softened, allowing the adaptation to the mouth of the patient while suitably maintaining the through channels 21 and the dispensing holes 18 pervious, and then preserving the acquired shape once cooled.

Selective closure means can also be provided for one or more dispensing holes 18, comprising for example caps, shutters, adhesive elements, or the like.

As mentioned above, in each of the embodiments of figures 6-15, the dispensing terminal 17 comprises an inlet mouth 19 that can be connected to the end 3a of the head 3 of the dispenser 2.

The connection between the parts can be direct, or with interposition of a cannula 6, e.g. deformable, in order to reach specific zones, or in any case to allow maintaining a certain distance between the dispensing terminal 17 and the dispenser 2, facilitating the maneuvers of the operator and the comfort of the patient.

A quick coupling connection can be provided between the aforesaid parts - e.g. bayonet connection or the like - in order to allow the operator to quickly replace the dispensing terminal 17, in relation to the specific requirements.

A morphofunctional study was carried out in order to evaluate the capacity of penetration of the gas-liquid mixture in the treated zones.

For this study, ten patients were engaged, of age comprised between 18 and 50 years old, which required a dental extraction; patients affected by situations that could interfere with the results, such as a past treatment with bisphosphonates (drugs which cause ulcers of the oral cavity) or tabagism, were excluded.

The patients were then divided into two groups and subjected to the treatment: one group treated with nebulized povidone-iodine by means of the concentrator C, and the second group subjected to the application of a sterile gauze soaked with povidone-iodine on the operation site.

Subsequently, a tissue sample was taken for the histological analysis and to verify the results.

The histological analysis of the alveolar mucous membrane shows a greater absorption of the povidone-iodine solution in patients treated with the device 1 according to the invention, with respect to that of the group treated with the application of the sterile gauze.

A clinical study was also carried out in order to evaluate the clinical effectiveness of the treatment.

For this study, twenty patients were engaged of age comprised between 20 and 40 years old, having moderate gingivitis induced by plaque.

Also in this study, patients were excluded who were in situations that could alter the results, such as antibiotic therapy during the preceding month or use of mouthwash with chlorhexidine.

Before treatment with the mixture enriched with oxygen and povidone-iodine, the patients were subjected to tests aimed to evaluate the presence of periodontitis (PSR), the gingival bleeding index (GBI) and the percentage of plaque present (PCR).

The treatment protocol provided for two sessions 4 days apart, while the above-described tests were carried out at the start, 4 days after the first session and 11 days after the second session.

The results show that the experimental treatment of oxygen therapy with povidone-iodine produces a drastic decrease of the marginal bleeding index between the initial stage and the two reevaluation times, with improvement of the clinical signs and of the gingivitis, in terms of reduction of edema and redness.

It was thus seen that the invention achieves the proposed purposes.

The device according to the invention allows carrying out the treatment of various pathologies of the oral cavity in a much more effective manner with respect to that known by the man skilled in the art, obtaining improved results in shorter times. The synergistic action of topical oxygen and povidone-iodine, in particular, has shown to be much more effective than the known treatments which comprise the simple spraying with oxygen or the local application of povidone-iodine, or other substances with similar properties, e.g. with gauze.

Indeed, oxygen deeply penetrates into the tissues and drives therewith the further molecules present in the nebulized liquid, and this allows reaching zones not reachable with the treatments of known type.

Therefore, the substances present in the liquid (mainly but not exclusively povidone-iodine) are used in quantities much lower than those used with other currently-known therapeutic techniques.

The aforesaid results are obtained with a simple and inexpensive solution from a structural and functional standpoint.

In particular, the treatment can be quickly carried out also due to the particular shape of the dispensing terminal of the device, which allows treating even quite extensive zones, in an accurate manner and in relatively reduced times.

If the technical characteristics mentioned in the enclosed claims are followed by reference numbers, such reference numbers are only introduced with the purpose of increasing the clarity of the claims, and consequently the aforesaid reference numbers have no limiting effect on the interpretation of each element identified as an example by such reference numbers.

The present invention was described according to preferred embodiments, but equivalent variants can be conceived without departing from the protective scope offered by the following claims.

## Claims

1. Medical device for intraoral use, comprising at least one oxygen concentrator (C) which is connected to at least one dispenser (2) of a gaseous mixture enriched with oxygen, said dispenser (2) comprising at least one dispensing head (3) adapted to be positioned in proximity to a pre-established zone of the surface of the oral cavity of the patient, **characterized in that** it comprises means (B) for dispensing at least one liquid within the gaseous mixture enriched with oxygen, so as to spray said pre-established zone with said gaseous mixture carrying said nebulized liquid, said liquid comprising one or more active principles having disinfectant, therapeutic and/or nutritional properties.

2. Device, according to claim 1, wherein said liquid comprises povidone-iodine and/or chlorhexidine.

3. Device, according to claim 1 or 2, wherein said liquid comprises vitamin complexes.

4. Device according to one of the preceding claims, wherein said dispensing head (3) comprises at least one dispensing terminal (17) comprising a dispensing surface (20) affected by a plurality of dispensing holes (18).

5. Device, according to claim 4, wherein said dispensing terminal (17) is substantially disc-shaped.

6. Device according to claim 4, wherein said dispensing terminal (17) is substantially plate-shaped.

7. Device, according to claim 4, wherein said dispensing terminal (17) is substantially shaped as a cylindrical crown portion, which extends for a specific opening angle (α).

8. Device according to the preceding claim, wherein said opening angle (α) is comprised between 60° and 120°.

9. Device according to claim 4, wherein said dispensing terminal (17) is shaped as a dental bite.

10. Device according to the preceding claim, wherein said dispensing terminal (17) comprises a first external wall (22) having at least one first edge (23), and a second internal wall (24) having at least one second edge (25), said dispensing holes (18) being provided on said first wall (22) and second wall (24).

11. Device according to the preceding claim, wherein said dispensing holes are provided on said first edge (23) and/or on said second edge (25).

12. Device according to one of claims 4-11, wherein said dispenser (2) comprises at least one cannula (6), rigid or deformable, for connection to said dispensing terminal (17).

13. Device according to one of claims 4-11, wherein said dispensing holes (18) are distributed over said dispensing surface (20) in a uniform manner, or they are more concentrated in specific zones of said dispensing surface (20).

14. Device according to one of the preceding claims, wherein said gaseous mixture enriched with oxygen, carrying said liquid therewith, is dispensed over said affected zones at a pressure comprised between 1 and 1.6 atmospheres.

15. Device, according to one of the preceding claims, wherein said dispensing means (B) comprise at least one tank (11) of said liquid, associated with said dispenser (2).
